(19) Europäisches Patentamt
European Patent Office
Office européen des brevets

(11) **EP 4 464 244 A1**

(12) **EUROPEAN PATENT APPLICATION**
published in accordance with Art. 153(4) EPC

(43) Date of publication:
**20.11.2024 Bulletin 2024/47**

(21) Application number: **23756568.4**

(22) Date of filing: **09.02.2023**

(51) International Patent Classification (IPC):
**A61B 5/11** (2006.01)       **A61B 5/00** (2006.01)

(52) Cooperative Patent Classification (CPC):
**A61B 5/00; A61B 5/11**

(86) International application number:
**PCT/KR2023/001898**

(87) International publication number:
**WO 2023/158155 (24.08.2023 Gazette 2023/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.02.2022 KR 20220019597**
       **01.04.2022 KR 20220041172**

(71) Applicant: **Samsung Electronics Co., Ltd
Suwon-si, Gyeonggi-do 16677 (KR)**

(72) Inventors:
• **CHOI, Changlim
  Suwon-si, Gyeonggi-do 16677 (KR)**
• **KIM, Changhoon
  Suwon-si, Gyeonggi-do 16677 (KR)**
• **BAE, Hyunjin
  Suwon-si, Gyeonggi-do 16677 (KR)**
• **CHOI, Siyoul
  Suwon-si, Gyeonggi-do 16677 (KR)**

(74) Representative: **Walaski, Jan Filip et al
Venner Shipley LLP
200 Aldersgate
London EC1A 4HD (GB)**

(54) **ELECTRONIC DEVICE FOR DETECTING DESIGNATED MOVEMENT AND METHOD THEREFOR**

(57)     An electronic device according to various embodiments of the present disclosure comprises: a motion sensor; a memory; and at least one processor operatively coupled to the memory and the motion sensor, wherein the processor may be configured to: calculate a vector on the basis of a sensor value received from the motion sensor; check changes in time of the x-axis angle and the y-axis angle of the vector; detect a start time and an end time of a designated movement on the basis of the change; and calculate exercise information on the basis of the start time and the end time.

FIG. 3

EP 4 464 244 A1

## Description

### [Technical Field]

Various embodiments of the disclosure relate to an electronic device and a method for detecting designated movement, for example, to an electronic device and a method for detecting designated movement of an electronic device and providing services based thereon.

### [Background Art]

In line with the development of technology, electronic devices are evolving to be smaller for easy carrying and to perform various functions in various forms of use depending on user needs. One of such devices is a wearable device that can be directly attached to a part of the user's body for use.

Nowadays various electronic devices including wearable devices are equipped with multiple sensors and are able to obtain various information and provide various services based on the obtained information.

### [Disclosure of Invention]

### [Technical Problem]

Various embodiments of the disclosure are intended to provide an electronic device for detecting a designated movement through at least one sensor mounted to the electronic device, and a method therefor.

Various embodiments of the disclosure are intended to provide an electronic device for detecting a designated movement through at least one sensor mounted to the electronic device and providing various services based on the same, and a method therefor.

The technical problems to be solved in the disclosure are not limited to the technical problems mentioned above, and other technical problems not mentioned will be clearly understood by those skilled in the art to which the disclosure pertains from the description below.

### [Solution to Problem]

An electronic device according to various embodiments of the disclosure may include a motion sensor, a memory, and at least one processor operatively connected to the memory and the motion sensor, wherein the processor may be configured to calculate a vector, based on sensor values received from the motion sensor, identify changes in an x-axis angle and a y-axis angle of the vector over time, detect a start time and an end time of a designated movement, based on the changes, and produce exercise information, based on the start time and the end time.

A method of an electronic device according to various embodiments of the disclosure may include calculating a vector, based on sensor values received from a motion sensor of the electronic device, identifying changes in an x-axis angle and a y-axis angle of the vector over time, detecting a start time and an end time of a designated movement, based on the changes, and producing exercise information, based on the start time and the end time.

### [Advantageous Effects of Invention]

According to various embodiments, the electronic device is able to detect a specified motion through at least one equipped sensor and extract various information related thereto.

According to various embodiments, the electronic device is able to detect a specified motion through at least one equipped sensor and extract various information related thereto, thereby providing a service.

In addition, various effects that are directly or indirectly identified through this document may be provided.

### [Brief Description of Drawings]

In relation to the description of the drawings, the same or similar reference numerals may be used for the same or similar components.

FIG. 1 is a block diagram of an electronic device in a network environment according to various embodiments.
FIG. 2 illustrates an example of the appearance of an electronic device for detecting a designated movement according to various embodiments.
FIG. 3 is a block diagram of an electronic device for detecting a designated movement according to various embodiments.

FIG. 4 is a diagram illustrating a designated-movement detection operation of an electronic device according to various embodiments.

FIG. 5 is a diagram illustrating a designated-movement detection operation of an electronic device in more detail according to various embodiments.

FIG. 6 is a diagram illustrating an example of a service providing operation according to detection of a designated movement by an electronic device according to various embodiments.

FIG. 7 is a diagram illustrating another example of a service providing operation according to detection of a designated movement by an electronic device according to various embodiments.

FIG. 8 is a flowchart illustrating a designated movement detection operation of an electronic device according to various embodiments.

**[Mode for the Invention]**

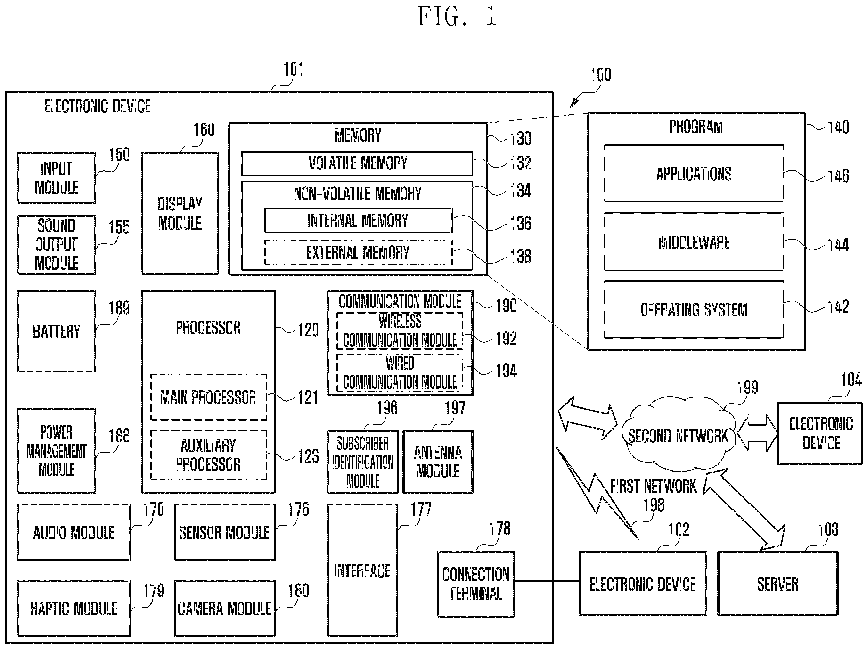

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

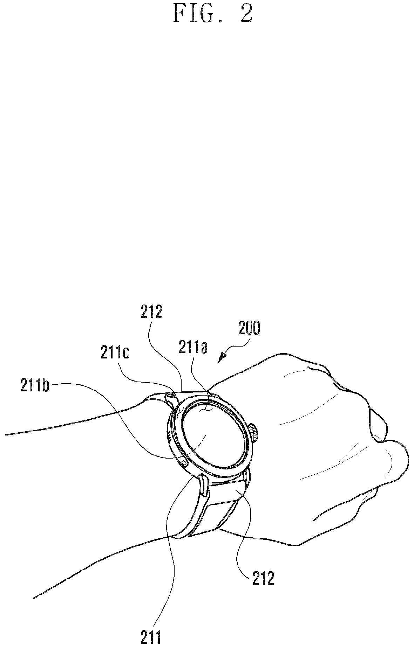

FIG. 2 illustrates the appearance of an electronic device 200 according to various embodiments.

Although the electronic device 200 in FIG. 2 (e.g., the electronic device 101 in FIG. 1) is illustrated as a wearable device worn on a user's wrist, this is only an example, and the electronic device 200 is not limited thereto, and may include various types of portable electronic devices. For example, the electronic device 200 may include a portable electronic device (e.g., a smartphone or a tablet PC) or a wearable device (e.g., a wireless audio device, a smart watch, or a lost-child prevention device) capable of short-range wireless communication according to at least two types of communication protocols. For example, the electronic device 200 may be implemented as various wearable devices, such as a body-attached device (e.g., a health patch or a digital tattoo), a clothing-type device (e.g., smart clothing or gloves), or a band-type device (e.g., a wrist/arm/finger band or a smart ring).

Referring to FIG. 2, when implemented as a wearable device, the electronic device 200 may include a housing 211 including a first surface (or front surface) 211a, a second surface (or rear surface) 211b, and a side surface 211c surrounding a space between the first surface 211a and the second surface 211b, and a fastening member 212 (e.g., a strap) connected to at least a portion of the housing 211 and configured to be detachably attached to a part of the user's body (e.g., a wrist or ankle).

According to various embodiments, the front surface 211a may be formed by a front plate (e.g., a glass plate or polymer plate including various coating layers) that is substantially transparent at least in part.

According to various embodiments, the second surface 211b may be formed by a rear plate that is substantially opaque. The rear plate may be formed of, for example, coated or tinted glass, ceramic, polymer, metal (e.g., aluminum, stainless steel (STS), or magnesium), or a combination of at least two of the above materials.

According to various embodiments, the side surface 211c may be formed by a side bezel structure (or "side member") partially coupled to the front plate (e.g., the first surface 211a) and the rear plate (e.g., the second surface 211b) and including metal and/or polymer.

According to various embodiments, the rear plate and the side bezel structure may be integrally formed and may include the same material (e.g., a metal material such as aluminum). The fastening member 212 may be formed of various materials and shapes. For example, it may be formed integrally or such that a plurality of unit links are movable relative to each other by a woven material, leather, rubber, urethane, metal, ceramic, or a combination of at least two of the above materials.

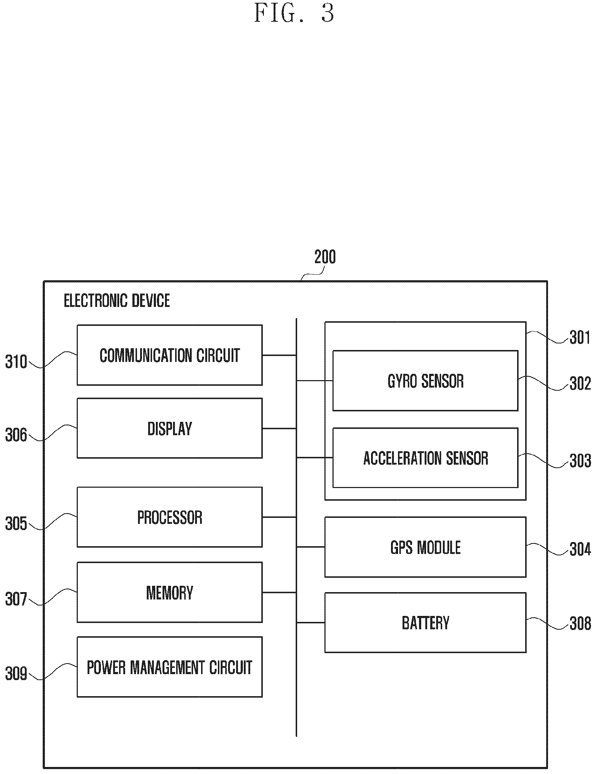

FIG. 3 is a block diagram of an electronic device (e.g., the electronic device 200 in FIG. 2) for detecting a designated movement according to various embodiments. The electronic device 200 in FIG. 3 may be implemented as a wearable device that may be worn on a user's body part (e.g., a wrist), as described with reference to FIG. 2.

Referring to FIG. 3, the electronic device 200 may include a motion sensor 301 (e.g., the sensor module 176 in FIG. 1), a global positioning system (GPS) module 304, a processor 305 (e.g., the processor 120 in FIG. 1), a touchscreen display 306 (e.g., the display device 160 in FIG. 1), a memory 307 (e.g., the memory 130 in FIG. 1), a battery 308 (e.g., the battery 189 in FIG. 1), a power management circuit 309 (e.g., the power management module 188 in FIG. 1), and a communication circuit 310 (e.g., the communication module 190 in FIG. 1). For example, the electronic device 200 may include at least some components that are identical or similar to the components of the electronic device 101 illustrated in FIG. 1. In addition, the electronic device 200 may exclude at least one of the components described above, or may further include one or more other components (e.g., a camera module).

According to an embodiment, the motion sensor 301 may also be called an inertial sensor or a six-axis sensor, and may include a gyro sensor 302 and an acceleration sensor 303. The acceleration sensor (accelerometer) may be a sensor that measures dynamic forces such as acceleration, vibration, and impact of an object. The acceleration sensor 303 may detect the motion state of an object, and thus may be utilized for various purposes. The electronic device 200 may detect a designated movement (e.g., a wheelchair pushing motion), based on acceleration data measured by the motion sensor 301. The gyro sensor (gyroscope) 302 may be a sensor that measures the angular velocity of the electronic device 200. The angular velocity measured by the gyro sensor 302 may denote a rotational speed (or angle) per hour. The gyro sensor 302 may also be referred to as a gyroscope.

According to an embodiment, the GPS module 304 may receive signals from GPS satellites, calculate the distance between the electronic device 200 and the satellites, based on the received GPS signals, and determine the location of the electronic device 200 as, for example, geographic coordinates or GPS coordinates expressed as a pair of latitude and longitude.

According to an embodiment, the processor 305 may control the overall operation of the electronic device 200 and the signal flow between the internal components of the electronic device 200, and may perform data processing.

According to an embodiment, the display 306 may be implemented as a touchscreen display including a touch sensor configured to detect a touch or a pressure sensor configured to measure the intensity of a force generated by the touch. The display 306 may visually display various information including images and/or text under the control of the processor 305. The display 306 may be implemented as one of, but not limited to, a liquid crystal display (LCD), a light-emitting diode (LED) display, an organic light-emitting diode (OLED) display, a micro-electro mechanical systems (MEMS) display, or an electronic paper display.

According to an embodiment, the display 306 may visually display various information including exercise information based on battery level information and/or movement-related information of the electronic device 200.

According to an embodiment, the memory 307 may store various data, applications, and/or programs for the processor 305 to control the overall operation of the electronic device 200, process various information, and provide various functions. For example, the memory 307 may store various data, applications and/or programs necessary or generated for the processor 305 to detect a designated movement, based on a sensor value sensed by controlling the motion sensor 301, obtain various exercise information, based on the detected movement-related information, and provide the same as visual information through, for example, the display 306.

According to an embodiment, the power management circuit 309 may control the power supply from the battery 308 (e.g., the battery 189 in FIG. 1) to the components of the electronic device 200 including the processor 305.

According to an embodiment, the communication circuit 310 may communicate with an external electronic device (e.g., the electronic device 102, the electronic device 104, and/or the server 108 in FIG. 1), based on a short-range communication network (e.g., the first network 198 in FIG. 1) through low-power short-range communication such as Bluetooth or Bluetooth low energy (BLE) and/or short-range communication (e.g., wireless fidelity (Wi-Fi), Wi-Fi direct, ultra-wideband (UWB), or hotspot), and a long-range communication network (e.g., the second network 199 in FIG. 1) such as a legacy cellular network, a 5G network, a next-generation communication network, the Internet, or a computer network (e.g., LAN or WAN).

In general, when a user rides a wheelchair, exercise information based on the number of steps is unable to be provided. According to an embodiment, the electronic device 200 may provide a wheelchair user with exercise information related to wheelchair use. To this end, the electronic device 200 may detect a designated movement for the forward movement of the wheelchair, for example, a motion of pushing wheelchair wheels with hands, and may produce and provide exercise information based on the same.

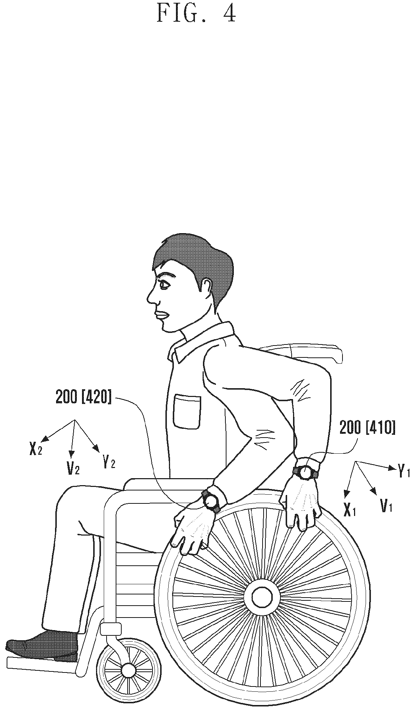

FIG. 4 is a diagram showing a motion in which a user wearing an electronic device 200 pushes a wheelchair according to various embodiments. Hereinafter, various embodiments will be described in detail with reference to FIGS. 3 and 4.

According to an embodiment, as the electronic device 200 moves due to the motion of the wheelchair user, the processor 305 of the electronic device 200 may receive a sensor value from the motion sensor 301 of the electronic device 200. The sensor value received from the motion sensor 301 may include, for example, angular velocity information received from the gyro sensor 302 and/or acceleration information received from the acceleration sensor 303. Hereinafter, an operation of producing exercise information, based on acceleration information received from the acceleration sensor 303 will be described in detail, but the embodiment is not limited thereto, and the exercise information may be obtained by calculating a rotation angle value from the angular velocity information received from the gyro sensor 302 or using it as additional information.

According to an embodiment, the processor 305 may calculate movement information, based on the sensor value of the motion sensor 301, and detect a designated movement, based on this. For example, the movement information may include acceleration information, and the acceleration information may be produced from a value obtained by measuring, using the acceleration sensor 303, information on a force applied to the electronic device 200 with respect to the gravitational acceleration. The processor 305 may obtain x-axis, y-axis, and z-axis components or angles of a vector V of the force acting on the electronic device 200 according to the tilt of the electronic device 200 from the acceleration information, respectively. The x-axis, y-axis, and z-axis components of the vector V may indicate the angle at which the x-axis is tilted, the angle at which the y-axis is tilted, and the angle at which the z-axis is tilted, respectively, with respect to the ground.

According to an embodiment, the processor 305 may detect a designated movement, based on changes in the x-axis component and y-axis component of the vector V.

According to an embodiment, the processor 305 may analyze the correlation between the changes in the x-axis component and y-axis component of the vector V and, if it shows a negative correlation, determine that a designated movement has occurred. For example, based on the correlation between changes in the x-axis angle and y-axis angle of a first vector calculated at a specific time and a second vector calculated at a different time, the start time and the end time of a period indicating a negative correlation may be detected as a start time and an end time of the designated movement. The change in the x-axis component and y-axis component of the vector V may be analyzed as the x-axis angle gradient and the y-axis angle gradient, respectively.

According to an embodiment, the processor 305 may detect the time at which the x-axis component change of the vector V switches from a value of 0 to a negative value and at which the y-axis component change switches from a value of 0 to a positive value as the time at which the designated movement starts.

When the user starts pushing the wheelchair, the processor 305 may detect changes in the x-axis angle and y-axis angle at the time [410], for example, that a change in the x-axis angle switches from a value of 0 to a negative value and that a change in the y-axis angle switches from a value of 0 to a positive value, and may determine that the designated movement starts.

According to an embodiment, the processor 305 may detect the time at which the x-axis component change of vector V switches from a negative value to a value of 0 and at which the y-axis component change switches from a positive value to a value of 0 as the time at which the designated movement ends.

When the user ends pushing the wheelchair, the processor 305 may detect the changes of the x-axis angle and y-axis angle at the time [420], for example, that the x-axis angle change switches from a negative value to a value of 0 and that the y-axis angle change switches from a positive value to a value of 0, and may determine that the designated movement ends.

In an embodiment, the processor 305 may further calculate the angular change between the vector (the first vector V1) at the start time [410] and the vector (the second vector V2) at the end time [420], and determine that the designated movement has occurred once if the calculated angular change is greater than or equal to a specified threshold value (e.g., 25 degrees). The processor 305 may detect the start time and end time of the designated movement and repeat the operation of calculating the angular change of the vector, thereby counting the number of occurrences of the designated movement.

According to an embodiment, if the start and end times of the designated movement are detected based on the sensor values of the sensor 301, the processor 305 may count that the designated movement has occurred once.

According to an embodiment, in the case where the start and end times of the designated movement are detected based on the sensor values of the sensor 301, the processor 305 may further count that the designated movement has occurred once if the angular change of the vector is equal to or greater than a specified threshold value.

According to an embodiment, various exercise information such as the speed, intensity, number of times, exercise amount, and movement distance of the designated movement may be calculated based on information such as the time interval between the start time and the end time of the designated movement, the angular change of the vector V, and/or the number of occurrences.

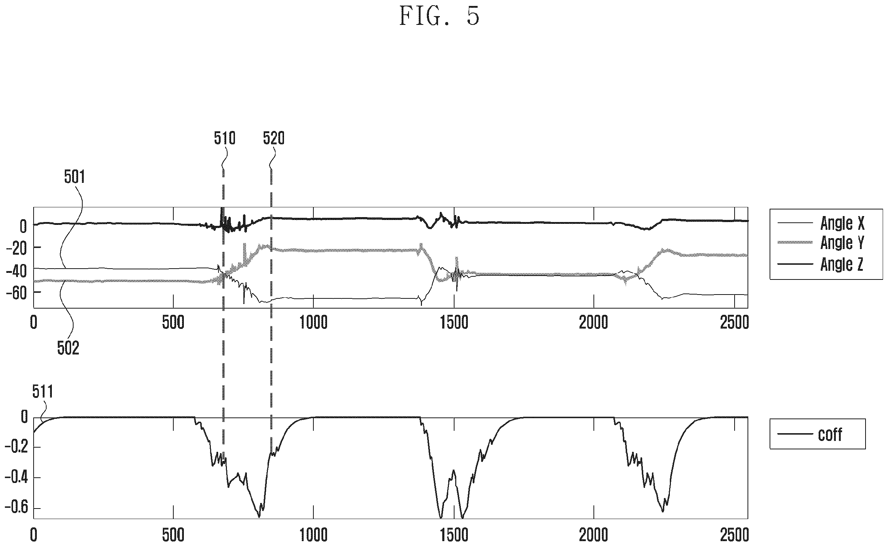

FIG. 5 is a diagram illustrating a designated-movement detection operation of an electronic device 200 in more detail according to various embodiments. FIG. 5 may represent the degree to which the x-axis component or angle 501 and the y-axis component or angle 502 of the vector V of force calculated based on the sensor values measured by the motion sensor 301 change along the time axis. In this case, it can be seen that the changes or slopes of both the x-axis angle 501 and the y-axis angle 502 show a value of 0 until the time 510 at which the designated movement starts, that the change or slope of the x-axis angle 501 switches from 0 to negative at the time 510, and that the change or slope of the y-axis angle 502 switches from 0 to positive. Therefore, the processor 305 of the electronic device 200 may detect that the designated movement starts at the time 510.

According to an embodiment, it can be seen that the change or slope of the x-axis angle 501 is negative and the change or slope of the y-axis angle 502 continues to be positive after the time 510, and that the change or slope of the x-axis angle 501 switches from negative to 0 and the change or slope of the y-axis angle 502 switches from positive to negative at the time 520. Therefore, the processor 305 of the electronic device 200 may detect that the designated movement is terminated at the time 520. Accordingly, the processor 305 may count one execution of the designated movement. In addition, the processor 305 may determine whether or not the designated movement occurs, based on the amount of change of a vector including the x-axis angle and the y-axis angle of the start time 510 and the end time 520 of the designated movement.

Referring to FIG. 5, it can be seen that a graph 511 showing the correlation between changes of the x-axis angle and y-axis angle indicates a negative correlation between the start time 510 and the end time 520 of the designated movement. The processor 305 of the electronic device 200 may analyze and monitor the correlation between changes of the x-axis angle and y-axis angle, thereby monitoring whether or not the designated movement occurs.

According to an embodiment, the correlation between changes of the x-axis angle and y-axis angle may be expressed by Equation 1 below.

[Equation 1]

$$x'=\frac{1}{n}\sum x_i, \ y'=\frac{1}{n}\sum y_i$$
$$S_{xx}=\sum(x_i-x')^2, \ S_{yy}=\sum(y_i-y')^2, \ S_{xy}=\sum(x_i-x')(y_i-y')$$
$$r=\frac{S_{xy}}{\sqrt{S_{xx}}\sqrt{S_{yy}}}$$

Here, $x_i \in \{x1, x2,..., x_n\}$ may represent the x-axis angle over time, $y_i \in \{y1, y2,..., y_n\}$ may represent the y-axis angle over time, and r may represent the correlation between changes of the x-axis angle and y-axis angle. In FIG. 5, while the correlation (value r) 511 indicates a negative value, it may be determined that, for example, a designated-movement event occurs between the time 510 and the time 520.

According to an embodiment, the x-axis angle slope and the y-axis angle slope start to switch just before the designated-movement event occurs as described above, and this time 510 may be determined as an event start time. In addition, after the designated-movement event start time, the x-axis angle slope and the y-axis angle slope start to switch again as described above, and this time 520 may be determined as an event end time.

In general, the x-axis angle and y-axis angle of the vector at the times at which the wheelchair pushing motion starts and ends may differ between wheelchair users. According to an embodiment, the designated-movement start time and end time for detecting the wheelchair pushing motion may be determined based on the time at which the slope of the x-axis angle and the slope of the y-axis angle switch or their correlation, and thus, the designated movement may be defined based on the change trend and/or correlation of the slope of the x-axis angle and the slope of the y-axis angle, thereby dynamically determining and detecting whether or not the designated movement occurs.

According to an embodiment, if the start time 510 and the end time 520 of the designated movement are detected, the processor 305 may calculate the angular change of the vector of the start time 510 and the end time 520.

Equation 2 below may represent an equation for calculating the change (e.g., angular velocity) of the vector.

[Equation 2]

$$\theta = \cos^{-1}\left(\frac{a_x b_x + a_x b_x + a_x b_x}{\sqrt{a_x^2 + a_y^2 + a_z^2}\sqrt{b_x^2 + b_y^2 + b_z^2}}\right)$$

Here, the first vector $a=(a_x, a_y, a_z)$ and the second vector $b=(b_x, b_y, b_z)$ may respectively represent vector values (x-axis component, y-axis component, z-axis component) at the start time and the end time, and $\theta$ may represent the amount of change in the vector between the start time and the end time.

According to an embodiment, the processor 305 may determine that the designated movement has occurred once if the amount of change of the vector is greater than or equal to a specified threshold value.

According to an embodiment, the processor 305 may determine the time interval between the start time 510 and the end time 520 of the designated movement as the time during which the designated movement, for example, the movement of pushing the wheelchair, continues.

According to an embodiment, the processor 305 may calculate the exercise intensity and/or exercise speed, based on the duration of the designated movement, the amount of angular change of the vector between the start time 510 and the end time 520, and/or the interval between the designated-movement occurrence events. For example, based on the value obtained by dividing the amount of angular change of the vector due to the occurrence of the designated movement by the duration, if the amount of angular change per time is large, it may be determined that the exercise intensity is high. For example, if the interval between the designated-movement occurrence events is short, it may be determined that the exercise intensity becomes higher as the exercise speed increases.

Equation 3 below may represent an equation for calculating the angular velocity obtained by dividing the angular change of the vector due to the occurrence of the designated movement by the duration of the relevant movement as the exercise intensity.

[Equation 3]

$$\theta' = \frac{\theta}{t_e - t_s}$$

Here, $\theta$ represents the amount of change of the vector between the start time and the end time in Equation 2, and $t_e$ represents the time of the end time and $t_s$ represents the time of the start time, which may be expressed, for example, in units of minutes. According to an embodiment, in addition to the amount of change per time (angular velocity) of the vector, the interval between occurrences of the designated-movement event (movement occurrence frequency) may be measured and the exercise intensity may be corrected by further reflecting this.

According to an embodiment, the calories burned may be calculated based on the number of occurrences of designated movement and/or the exercise intensity. For example, when a designated-movement event occurs, the calories burned may be calculated based on the exercise intensity for each event. For example, the calories burned may be calculated based on the exercise intensity for the entire counted movement events and further based on information about the event occurrence interval. For example, the exercise intensity and/or the event occurrence interval may be applied as weights when calculating the calories burned.

According to an embodiment, the processor 305 may calculate additional exercise information through the pattern of the detected designated movement. For example, if a non-periodic but continuous movement is detected, the processor 305 may determine the same as general wheelchair driving. For example, if a periodic and continuous movement is detected, the processor 305 may determine the same as wheelchair driving for exercise. For example, if a periodic and short-cycle and/or high-intensity movement is repeated, the processor 305 may determine the same as uphill wheelchair driving, and in this case, the weight may be further reflected in the calculation of the calories burned depending on the length of the cycle.

According to an embodiment, the processor 305 may estimate, for example, the movement distance, based on the number of detections and/or the intensity of the designated movement. For example, the movement distance will be proportional to the number of detections of movement and may also be proportional to the intensity. However, on the flat ground, the amount of rotation of the wheelchair wheel will be greater than the occurrences of the designated movement, and on an uphill slope, the amount of rotation of the wheelchair wheel will be less than the occurrences of the designated movement. Therefore, the movement distance may be calculated in consideration of the duration, intensity, number of detections, and/or detection cycle of the designated movement.

According to an embodiment, the processor 305 may identify the movement distance, based on position values received from the GPS module 304. In this case, the processor 305 may compare the movement distance based on the GPS position values with the designated-movement detection cycle and, if the movement distance for one movement event detection is less than the circumference of the wheels of the wheelchair (a value obtained by multiplying the wheelchair radius by $2\pi$), may determine it as an uphill slope and, if it is more than twice the circumference of the wheels of the wheelchair, determine it as a downhill slope.

According to an embodiment, in the case where a sensor capable of detecting rotation is attached to the wheelchair, the processor 305, if the rotation angle of the wheelchair for each detection of the designated movement is less than, for example, 180 degrees, may determine it as an uphill slope and, if the rotation angle of the wheelchair is 720 degrees or more, determine it as a downhill slope. In this case, the processor 305 may further improve the reliability of the designated movement detection by utilizing sensor values of the sensor attached to the wheelchair, in addition to sensor values of the motion sensor 301. For example, if the rotation of the wheelchair wheel is detected at the time at which the processor 305 detects the designated movement, the reliability of the designated-movement detection may be further improved.

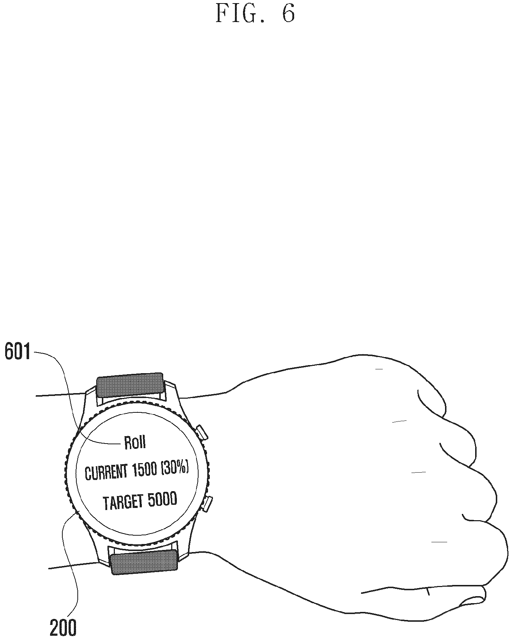

FIG. 6 is a diagram illustrating an example of a service providing operation according to detection of a designated movement by an electronic device 200 according to various embodiments.

According to an embodiment, the processor 305 of the electronic device 200 may detect a designated movement, based on sensor values as described above and produce various exercise information based on the same.

According to an embodiment, the electronic device 200 may perform a service of producing and providing exercise information. As a program providing wheelchair-related exercise information is activated, the processor 305 of the electronic device 200 may receive sensor values through the motion sensor 301, detect a designated movement based on the same, produce various exercise information, based on the detected movement, and provide it through the display 306.

Referring to FIG. 6, the electronic device 200 may display exercise information 601 on the display 306. Here, Roll may indicate the number of times the designated movement is detected according to the pushing motion for the wheelchair. For example, the number of times (Roll) the designated movement is detected may be represented as exercise information 601, and the number of times (e.g., 1500 times) the current Roll has been performed may be indicated compared to the target Roll (e.g., set to 5000 times), which indicates the amount of exercise corresponding to 30% of the target Roll. The exercise information 601 capable of being displayed on the display 306 is not limited thereto, and for example, exercise time may be displayed, and in this case, information indicating the current exercise time (e.g., 3 hours) compared to the target exercise time (e.g., 10 hours) and the exercise time percentage (e.g., 30 percent) compared to the target time.

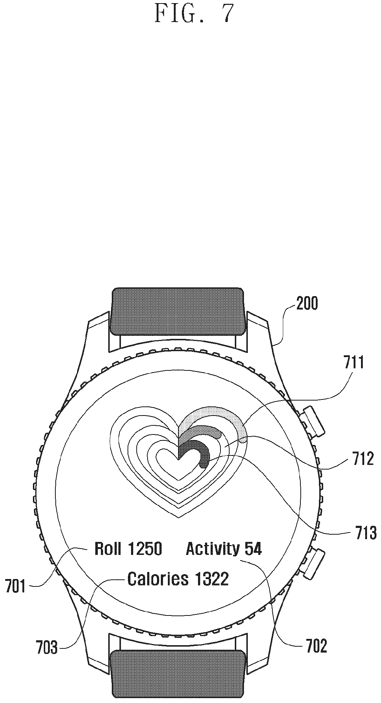

FIG. 7 is a diagram illustrating another example of a service providing operation according to detection of a designated movement by an electronic device 200 according to various embodiments.

According to an embodiment, the processor 305 of the electronic device 200 may detect a designated movement based on sensor values and produce various exercise information based on the same, as described above.

According to an embodiment, the electronic device 200 may perform a service of producing and providing exercise information. As a program providing wheelchair-related exercise information is activated, the processor 305 of the electronic device 200 may receive sensor values through the motion sensor 301, detect a designated movement based on the same, produce various exercise information based on the detected movement, and provide it through the display 306.

Referring to FIG. 7, the electronic device 200 may display various exercise information 701, 702, and 703 on the display 306. Here, Roll 701 may represent the number of times a designated movement is detected according to the pushing motion for the wheelchair, and for example, Roll 701 may indicate the number of times the designated movement is detected, i.e., the number of times of exercise.

Activity 702 may represent exercise time (e.g., in minutes). The exercise time may be calculated by summating the time for movement indoors or outdoors. For example, indoors, the exercise time may be determined as 1 minute from the time at which the designated movement is detected, and if the designated movement further occurs within 1 minute (e.g., after 20 seconds), the total exercise time may be 1 minute and 20 seconds, excluding the overlapping time. For example, outdoors, the exercise time may include the time at which movement is detected through the GPS module 304, in addition to the time at which the designated movement is detected. Meanwhile, the exercise time may be weighted depending on the exercise intensity (e.g., the intensity of the pushing motion). For example, the exercise time may be configured to be further accumulated when performing a high-intensity motion.

Calories 703 may represent the calories burned during the exercise time. For example, the calories 703 may be calculated, based on information such as the number of times exercised, exercise speed, and exercise intensity, in addition to the exercise time. For example, the processor 305 may produce information such as the number of times exercised and exercise intensity when detecting the designated movement, and calculate and provide calories burned up to the current time, compared to the recommended daily calories burned, as a basal metabolic rate and additional calories burned of the wheelchair user. Here, the number of times exercised may indicate the number of times the designated movement is detected. The exercise speed indicates the speed at which the designated movement is performed, and may be calculated based on the frequency at which the designated movement occurs during the entire exercise time. The exercise intensity may be calculated, when the designated movement is detected, as the amount of change (angular velocity) of the vector for the time during which the designated movement is performed. The exercise intensity may be determined by reflecting the exercise speed in addition to the angular velocity.

According to an embodiment, the processor 305 may display images 711, 712, and 713 that visually represent exercise information on the display 306. The images 711, 712, and 713 may represent various shapes including a heart shape, and may be provided in a form configured such that the images are filled with different colors depending on the amount of exercise information, enabling the exercise information to be easily and quickly recognized visually. For example, the first image 711 may represent the number of times exercised 701, and may be filled with different colors proportional to the current number of times exercised, relative to the entire shape image representing the recommended number of times exercised or the predetermined total number of times exercised, thereby visually expressing the current number of times exercised. For example, the second image 712 may indicate the exercise time 702, and may be filled with different colors proportional to the current exercise time performed, relative to the entire shape image representing the recommended exercise time or the predetermined entire exercise time, thereby visually expressing the current exercise time. For example, the third image 713 may indicate exercise calories burned 703, and may be filled with different colors proportional to the current calories burned by exercise, relative to the entire shape image representing the recommended calories burned or the predetermined total exercise calories burned, thereby visually expressing the number or level of the current exercise calories.

According to an embodiment, the processor 305 may provide a service of recommending exercise through sound, vibration, and/or visual notification if a designated movement is not detected for a specified time or longer, based on the exercise information. In this case, the exercise goal of the wheelchair user may be set in advance, based on the number of designated movements or various exercise information, and information about insufficient exercise may be provided based on this, and an alarm for recommending exercise may be provided.

An electronic device (e.g., the electronic device 200 in FIG. 2 or FIG. 3) according to various embodiments may include a motion sensor (e.g., the motion sensor 301 in FIG. 3), a memory (e.g., the memory 307 in FIG. 3), and at least one processor (e.g., the processor 305 in FIG. 3) operatively connected to the memory and the motion sensor, wherein the processor may be configured to calculate a vector, based on sensor values received from the motion sensor, identify changes in an x-axis angle and a y-axis angle of the vector over time, detect a start time and an end time of a designated movement, based on the changes, and produce exercise information, based on the start time and the end time.

According to an embodiment, the processor may detect a time at which the change in the x-axis angle of the vector switches from a value of 0 to a negative value and at which the change in the y-axis angle of the vector switches from a value of 0 to a positive value as the start time.

According to an embodiment, the processor may detect a time at which the change in the x-axis angle of the vector switches from a negative value to a value of 0 and at which the change in the y-axis angle of the vector switches from a positive value to a value of 0 as the end time.

According to an embodiment, the processor may detect, as the start time and the end time, a start time and an end time of a period indicating a negative correlation, based on a correlation between the changes in the x-axis angle and the y-axis angle of a first vector calculated at the start time and a second vector calculated at the end time.

According to an embodiment, the processor may calculate the amount of change of a first vector calculated at the start time and a second vector calculated at the end time and, if the change is equal to or greater than a specified threshold value, determine that the designated movement has occurred once.

According to an embodiment, the processor may calculate an angular change between the first vector and the second vector for the time interval between the start time and the end time as exercise intensity information.

According to an embodiment, the processor may accumulate the number of times the designated movement is detected as the exercise information and calculate calories burned, based on the number of accumulations and the exercise intensity.

According to an embodiment, the processor may add exercise time if the designated movement is continuously detected at a specified time interval or less and calculate calories burned by further considering the detection frequency of the designated movement in the exercise time.

According to an embodiment, the electronic device may further include a display, and the processor may display a visual image representing the exercise information as relative information to reference exercise information on the display.

According to an embodiment, the processor may provide a notification if the exercise information is relatively insufficient, compared to the reference exercise information.

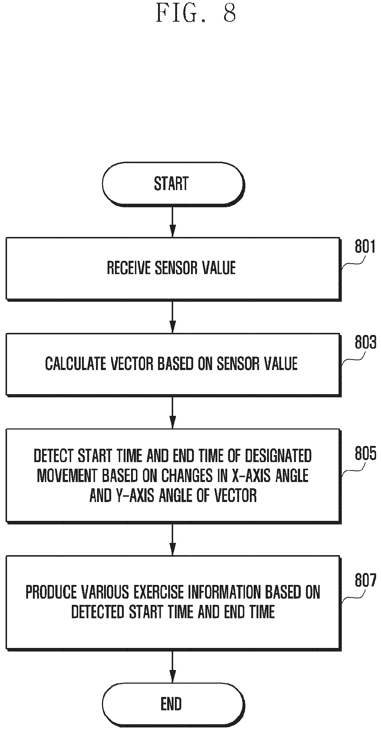

FIG. 8 is a flowchart illustrating a designated-movement detection operation of an electronic device (e.g., the electronic device 200 in FIG. 2, FIG. 3, or FIG. 4) according to various embodiments.

According to an embodiment, as a program providing exercise information is activated, the processor 305 of the electronic device 200 may receive sensor values from the motion sensor 301 of the electronic device 200 in operation 801. The sensor values received from the motion sensor 301 may include, for example, angular velocity information received from the gyro sensor 302 and/or acceleration information received from the acceleration sensor 303.

According to an embodiment, the processor 305 may produce movement information, for example, as a vector, based on the sensor values of the motion sensor 301 in operation 803. For example, the movement information may be produced as a vector V by extracting angle information for the x-axis, y-axis, and z-axis, based on the sensor values.

According to an embodiment, the processor 305 may detect a start time and an end time of the designated movement, based on changes in the x-axis angle and y-axis angle of the vector V in operation 805.

According to an embodiment, the processor 305 may analyze the correlation between the changes in the x-axis angle and y-axis angle of the vector V and, if it approaches a negative correlation, determine that a designated movement has occurred.

According to an embodiment, the processor 305 may detect the time at which the x-axis angle change of the vector V switches from a value of 0 to a negative value and at which the y-axis angle change switches from a value of 0 to a positive value as the designated-movement start time.

According to an embodiment, the processor 305 may detect the time at which the x-axis angle change of vector V switches from a negative value to a value of 0 and at which the y-axis angle change switches from a positive value to a value of 0 as the designated-movement end time.

According to an embodiment, if the start time and the end time of the designated movement are detected, the processor 305 may calculate the angular change between the vector at the start time and the vector at the end time and, if the calculated angular change is equal to or greater than a specified threshold value (e.g., 25 degrees), determine that the designated movement has occurred once.

According to an embodiment, the processor 305 may produce various exercise information, based on the start time and end time of the designated movement in operation 807.

According to an embodiment, the processor 305 may count the number of occurrences of the designated movement by repeating operations 803 and 805 of calculating a vector and detecting the start time and end time of the designated movement over time.

According to an embodiment, the processor 305 may count that the designated movement has occurred once when the start and end of the designated movement are detected by the sensor 301.

According to an embodiment, the processor 305 may produce various exercise information such as the speed, intensity, number of times, exercise amount, and movement distance of the designated movement by utilizing information such as the time interval between the start time and the end time of the designated movement, the amount of angular change of the vector V, and/or the number of times the designated movement is detected.

According to an embodiment, the processor 305 may calculate the time interval between the start time and the end time of the designated movement as the duration of the designated movement, for example, the motion of pushing a wheelchair.

According to an embodiment, the processor 305 may calculate the amount of angular change of the vectors between the start time and the end time for the designated-movement duration as exercise intensity information. For example, based on the value obtained by dividing the amount of angular change between the start time and the end time for the detected designated movement by the movement duration, if the amount of angular change per time is large, it may be determined that the exercise intensity is high.

According to an embodiment, the exercise speed may be calculated based on the interval between the designated-movement detection times. For example, if the interval of a specified designated-movement occurrence event is short, the exercise speed may increase, and if the interval is long, the exercise speed may decrease. The exercise speed may be used as additional information for determining the exercise intensity. For example, if the exercise speed is high, it may be determined that the exercise intensity is high.

According to an embodiment, the calories burned may be calculated based on the number of times the designated movement is detected, the exercise speed, and/or the exercise intensity. For example, when a designated movement is detected, the calories burned may be calculated based on the exercise intensity for each movement. For example, the calories burned based on each exercise intensity may be calculated based on the exercise intensity for each of the total counted movement detections and further based on exercise speed information according to the detection interval.

In an embodiment, the calories burned may be adjusted based on the pattern of the movement. For example, when going uphill, the calories burned may be weighted to increase compared to when going downhill or on the flat ground.

In an embodiment, the processor 305 may produce additional exercise information through the pattern of the detected movement. For example, when a non-periodic but continuous movement is detected, the processor 305 may determine that it is general wheelchair driving. For example, when a periodic and continuous movement is detected, the processor 305 may determine that it is wheelchair driving for exercise. For example, if a periodic and short-cycle and/or high-intensity movement is repeated, the processor 305 may determine that it is uphill wheelchair driving, and in this case, the weight may be further reflected in the calculation of the calories burned, based on the length of the cycle.

In an embodiment, the processor 305 may produce various exercise information, based on the number of times, duration, exercise intensity, and/or detection cycle of the designated movement. For example, the movement distance may be proportional to the number of times the movement is detected and may also be proportional to the intensity. However, on the flat ground, the amount of rotation of the wheelchair wheel will be greater than the occurrences of the designated movement, and on an uphill slope, the amount of rotation of the wheelchair wheel will be less than the occurrences of the designated movement.

According to an embodiment, the processor 305 of the electronic device 200 may detect the designated movement, based on the sensor values, as described above and provide various exercise information produced based on the detected movement through, for example, the display 306.

According to an embodiment, the processor 305 may display the various exercise information produced on the display 306 using various texts and/or images. The display 306 images illustrated in FIGS. 6 and 7 are examples of an operation of providing a service according to detection of the designated movement of the electronic device 200 according to various embodiments, and the disclosure is not limited thereto.

The designated-movement detection method described above with reference to FIG. 8 is not limited to the above-described description, and the sequence of the respective operations may vary, or specific operations may be omitted, or the operations may be combined in various orders, or other operations may be added thereto.

According to various embodiments, a method of an electronic device (e.g., the electronic device 200 in FIG. 2 or FIG. 3) may include calculating a vector, based on sensor values received from a motion sensor of the electronic device, identifying changes in an x-axis angle and a y-axis angle of the vector over time, detecting a start time and an end time of a designated movement, based on the changes, and producing exercise information, based on the start time and the end time.

According to an embodiment, the detecting may include detecting a time at which the change in the x-axis angle of the vector switches from a value of 0 to a negative value and at which the change in the y-axis angle of the vector switches from a value of 0 to a positive value as the start time.

According to an embodiment, the detecting may include detecting a time at which the change in the x-axis angle of the vector switches from a negative value to a value of 0 and at which the change in the y-axis angle of the vector switches from a positive value to a value of 0 as the end time.

According to an embodiment, the detecting may include detecting, as the start time and the end time, a start time and an end time of a period indicating a negative correlation, based on a correlation between the changes in the x-axis angle and the y-axis angle of a first vector calculated at the start time and a second vector calculated at the end time.

According to an embodiment, the producing of the exercise information may include calculating the amount of change of a first vector calculated at the start time and a second vector calculated at the end time and, if the change is equal to or greater than a specified threshold value, determine that the designated movement has occurred once.

According to an embodiment, the producing of the exercise information may include calculating an angular change between the first vector and the second vector for the time interval between the start time and the end time as exercise intensity information.

According to an embodiment, the producing of the exercise information may include may include accumulating the number of times the designated movement is detected as the exercise information and calculating calories burned, based on the number of accumulations and the exercise intensity.

According to an embodiment, the producing of the exercise information may include adding exercise time if the designated movement is continuously detected at a specified time interval or less and calculating calories burned by further considering the detection frequency of the designated movement in the exercise time.

According to an embodiment, the method may further include displaying a visual image representing the exercise information as relative information to reference exercise information.

According to an embodiment, the displaying may include providing a notification if the exercise information is relatively insufficient, compared to the reference exercise information.

The embodiments of the disclosure are merely presented as examples to easily explain and help understanding the technical content, and are not intended to limit the scope of the disclosure. Therefore, the scope of the disclosure should be interpreted to include all changes or modifications derived from the technical ideas of various embodiments of the disclosure, in addition to the embodiments disclosed herein.

**Claims**

1. An electronic device comprising:

   a motion sensor;
   a memory; and
   at least one processor operatively connected to the memory and the motion sensor,
   wherein the processor is configured to:

   calculate a vector, based on sensor values received from the motion sensor;
   identify changes in an x-axis angle and a y-axis angle of the vector over time;
   based on the changes, detect a start time and an end time of a designated movement; and
   based on the start time and the end time, produce exercise information.

2. The electronic device of claim 1,
   wherein the processor is configured to detect a time at which the change in the x-axis angle of the vector switches from a value of 0 to a negative value and at which the change in the y-axis angle of the vector switches from a value of 0 to a positive value as the start time.

3. The electronic device of claim 1,
   wherein the processor is configured to detect a time at which the change in the x-axis angle of the vector switches from a negative value to a value of 0 and at which the change in the y-axis angle of the vector switches from a positive value to a value of 0 as the end time.

4. The electronic device of claim 1,
   wherein the processor is configured to detect, as the start time and the end time, a start time and an end time of a period indicating a negative correlation, based on a correlation between the changes in the x-axis angle and the y-axis angle of a first vector calculated at the start time and a second vector calculated at the end time.

5. The electronic device of claim 1,
   wherein the processor is configured to calculate the amount of change of a first vector calculated at the start time and a second vector calculated at the end time and, if the change is equal to or greater than a specified threshold value, determine that the designated movement has occurred once.

6. The electronic device of claim 5,
   wherein the processor is configured to calculate an angular change between the first vector and the second vector for the time interval between the start time and the end time as exercise intensity information.

7. The electronic device of claim 6,
   wherein the processor is configured to accumulate the number of times the designated movement is detected as the exercise information and calculate calories burned, based on the number of accumulations and the exercise intensity.

8. The electronic device of claim 7,
   wherein the processor is configured to add exercise time if the designated movement is continuously detected at a specified time interval or less and calculate calories burned by further considering the detection frequency of the designated movement in the exercise time.

9. The electronic device of claim 1,

   further comprising a display,
   wherein the processor is configured to display a visual image representing the exercise information as relative information to reference exercise information on the display.

10. The electronic device of claim 9,
    wherein the processor is configured to provide a notification if the exercise information is relatively insufficient, compared to the reference exercise information.

11. A method of an electronic device, the method comprising:

calculating a vector, based on sensor values received from a motion sensor of the electronic device;
identifying changes in an x-axis angle and a y-axis angle of the vector over time and detecting a start time and an end time of a designated movement, based on the changes; and
producing exercise information, based on the start time and the end time.

12. The method of claim 11,
wherein the detecting comprises detecting a time at which the change in the x-axis angle of the vector switches from a value of 0 to a negative value and at which the change in the y-axis angle of the vector switches from a value of 0 to a positive value as the start time.

13. The method of claim 11,
wherein the detecting comprises detecting a time at which the change in the x-axis angle of the vector switches from a negative value to a value of 0 and at which the change in the y-axis angle of the vector switches from a positive value to a value of 0 as the end time.

14. The method of claim 11,
wherein the detecting comprises detecting, as the start time and the end time, a start time and an end time of a period indicating a negative correlation, based on a correlation between the changes in the x-axis angle and the y-axis angle of a first vector calculated at the start time and a second vector calculated at the end time.

15. The method of claim 11,
wherein the producing of the exercise information comprises calculating the amount of change of a first vector calculated at the start time and a second vector calculated at the end time and, if the change is equal to or greater than a specified threshold value, determine that the designated movement has occurred once.

# FIG. 1

EP 4 464 244 A1

FIG. 2

# FIG. 3

ELECTRONIC DEVICE — 200

| | |
|---|---|
| COMMUNICATION CIRCUIT — 310 | GYRO SENSOR — 301, 302 |
| DISPLAY — 306 | ACCELERATION SENSOR — 303 |
| PROCESSOR — 305 | GPS MODULE — 304 |
| MEMORY — 307 | BATTERY — 308 |
| POWER MANAGEMENT CIRCUIT — 309 | |

FIG. 4

200 [420]

$X_2$

$V_2$  $Y_2$

200 [410]

$Y_1$

$X_1$  $V_1$

FIG. 5

EP 4 464 244 A1

FIG. 6

601

Roll

CURRENT 1500 (30%)

TARGET 5000

200

FIG. 7

Roll 1250  Activity 54
Calories 1322

FIG. 8

```
                    ┌─────────────┐
                    │    START    │
                    └──────┬──────┘
                           │
                           ▼
        ┌──────────────────────────────────────┐
        │         RECEIVE SENSOR VALUE          │ ─ 801
        └──────────────────┬───────────────────┘
                           │
                           ▼
        ┌──────────────────────────────────────┐
        │  CALCULATE VECTOR BASED ON SENSOR VALUE│ ─ 803
        └──────────────────┬───────────────────┘
                           │
                           ▼
        ┌──────────────────────────────────────┐
        │ DETECT START TIME AND END TIME OF      │
        │ DESIGNATED MOVEMENT BASED ON CHANGES   │ ─ 805
        │ IN X-AXIS ANGLE AND Y-AXIS ANGLE OF    │
        │ VECTOR                                 │
        └──────────────────┬───────────────────┘
                           │
                           ▼
        ┌──────────────────────────────────────┐
        │ PRODUCE VARIOUS EXERCISE INFORMATION   │ ─ 807
        │ BASED ON DETECTED START TIME AND END   │
        │ TIME                                   │
        └──────────────────┬───────────────────┘
                           │
                           ▼
                    ┌─────────────┐
                    │     END     │
                    └─────────────┘
```

## INTERNATIONAL SEARCH REPORT

| | International application No. |
|---|---|
| | **PCT/KR2023/001898** |

**A. CLASSIFICATION OF SUBJECT MATTER**

**A61B 5/11**(2006.01)i; **A61B 5/00**(2006.01)i

According to International Patent Classification (IPC) or to both national classification and IPC

**B. FIELDS SEARCHED**

Minimum documentation searched (classification system followed by classification symbols)

A61B 5/11(2006.01); A61B 5/00(2006.01); A61B 5/0245(2006.01); A61B 5/16(2006.01); A63B 69/00(2006.01); A63B 71/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 모션센서(motion sensor), 벡터(vector), 시간(time), 변화(variation), 운동 (movement), 산출(calculation)

**C. DOCUMENTS CONSIDERED TO BE RELEVANT**

| Category* | Citation of document, with indication, where appropriate, of the relevant passages | Relevant to claim No. |
|---|---|---|
| X | JP 2012-000211 A (HITACHI LTD.) 05 January 2012 (2012-01-05)<br>See paragraphs [0049]-[0072]; and claims 1, 11 and 20. | 1-6,9,11-15 |
| Y | | 7,8,10 |
| Y | KR 10-2018-0047654 A (SAMSUNG ELECTRONICS CO., LTD.) 10 May 2018 (2018-05-10)<br>See paragraph [0090]. | 7,8 |
| Y | JP 2001-218745 A (NAGANO NATIONAL COLLEGE OF TECHNOLOGY) 14 August 2001 (2001-08-14)<br>See claim 1. | 10 |
| A | JP 2018-008015 A (CASIO COMPUT CO., LTD.) 18 January 2018 (2018-01-18)<br>See entire document. | 1-15 |

| ☑ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 May 2023** | **09 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/001898**

| C. | DOCUMENTS CONSIDERED TO BE RELEVANT | | |
|---|---|---|---|
| Category* | Citation of document, with indication, where appropriate, of the relevant passages | | Relevant to claim No. |
| A | KR 10-2021-0108275 A (SAMSUNG ELECTRONICS CO., LTD.) 02 September 2021 (2021-09-02)<br>See entire document. | | 1-15 |

Form PCT/ISA/210 (second sheet) (July 2022)

**INTERNATIONAL SEARCH REPORT**
Information on patent family members

International application No.

**PCT/KR2023/001898**

| Patent document cited in search report | | | Publication date (day/month/year) | Patent family member(s) | | | Publication date (day/month/year) |
|---|---|---|---|---|---|---|---|
| JP | 2012-000211 | A | 05 January 2012 | JP | 5557610 | B2 | 23 July 2014 |
| KR | 10-2018-0047654 | A | 10 May 2018 | CN | 107997767 | A | 08 May 2018 |
| | | | | CN | 107997767 | B | 16 August 2022 |
| | | | | EP | 3316259 | A1 | 02 May 2018 |
| | | | | US | 10702190 | B2 | 07 July 2020 |
| | | | | US | 2018-0116561 | A1 | 03 May 2018 |
| JP | 2001-218745 | A | 14 August 2001 | JP | 3421738 | B2 | 30 June 2003 |
| JP | 2018-008015 | A | 18 January 2018 | CN | 107545229 | A | 05 January 2018 |
| | | | | JP | 6862875 | B2 | 21 April 2021 |
| | | | | US | 2018-0007277 | A1 | 04 January 2018 |
| KR | 10-2021-0108275 | A | 02 September 2021 | US | 2021-0260441 | A1 | 26 August 2021 |
| | | | | WO | 2021-172843 | A1 | 02 September 2021 |

Form PCT/ISA/210 (patent family annex) (July 2022)